# EUROPÄISCHE PATENTSCHRIFT

(11) **EP 0 511 466 B1**
(45) Veröffentlichungstag und Bekanntmachung des Hinweises auf die Patenterteilung: **02.08.1995**
(21) Anmeldenummer: 92103006.0
(22) Anmeldetag: 22.02.1992
(51) Int. Cl.: A61K 7/48, A61K 7/50

(54) **Wässrige Tensidzubereitungen mit erhöhter Viskosität**
Aqueous surfactants preparations with an increased viscosity
Réparations aqueuses de tensioactives à viscosité augmentée

(30) Priorität: 30.04.1991 DE 4114141
(43) Veröffentlichungstag der Anmeldung: 04.11.1992
(73) Patentinhaber: HÜLS AKTIENGESELLSCHAFT, 45764 Marl (DE)
(72) Erfinder: Balzer, Dieter, Dr., W-4358 Haltern (DE)

(56) Entgegenhaltungen:
- EP-A- 0 384 983
- EP-A- 0 388 810
- EP-A- 0 408 965
- WO-A-91/14761

## Beschreibung

Die Erfindung betrifft wäßrige, fremdelektrolytarme Tensidzubereitungen mit erhöhter Viskosität zum Einsatz insbesondere in kosmetischen Formulierungen wie z. B. Shampoos, Bade- und Duschpräparaten, Handwaschpasten, Lotionen, etc.

Höhere Viskositäten sind in vielen Fällen wünschenswert. Sie erleichtern die Handhabung und gestatten eine einfache Dosierung. Enthält die Zubereitung eine zweite Phase (fest oder flüssig), so vermittelt die erhöhte Viskosität auch höhere Lagerstabilität. Ferner sind aus Marketinggründen höhere Viskositäten wäßriger Tensidzubereitungen willkommen, da der Verbraucher dünnflüssige Präparate häufig mit geringer Wirkstoffkonzentration assoziiert.

Wäßrige Tensidzubereitungen für den o. a. Anwendungszweck enthalten als Hauptkomponente meist Aniontenside wie insbesondere Fettalkoholethersulfate, Fettalkoholsulfate, Fettalkoholsulfosuccinate, Alkansulfonate, Ethercarbonsäuren, etc. zuweilen in Kombination mit Betainen, Ampholyten, Fettsäurealkanolamiden, etc. Eine einfache Viskositätserhöhung durch zugesetzte wasserlösliche anorganische Salze (Fremdelektrolyte) wie NaCl, NH₄Cl, Na₂SO₄, etc. ist hierbei letztlich nur bei Fettalkoholether- und Fettalkoholsulfaten und bei Eiweiß-Fettsäure-Kondensaten eventuell in Kombination mit amphoteren oder zwitterionischen Tensiden möglich (WO 91/14761, Stand der Technik gemäß Art. 54(3) EP), wobei die Elektrolytmengen meist recht hoch liegen müssen, was gewöhnlich unerwünscht ist, da die Elektrolyte in höherer Konzentration hautirritierend wirken. Viele andere insbesondere wegen ihrer hohen Haut- bzw. Schleimhautverträglichkeit für den o. a. Anwendungszweck besonders interessante Tenside lassen sich durch Fremdelektrolyte nicht oder nur sehr unzureichend verdicken.

Eine gewisse Alternative als Konsistenzregulator stellen Fettsäurealkanolamide dar, doch sind diese in kosmetischen Formulierungen zunehmend unerwünscht, da ein geringer Gehalt an freiem Alkanolamin als Nebenprodukt Anlaß zur Bildung von Nitrosamin sein kann. Gesucht wird daher nach stickstofffreien Additiven anstelle der Alkanolamide (vgl. H. Hensen et al., 2ter Welt-Tensid-Kongreß, Paris 1988, Vol. II, 378 ff.).

Eine mit Nachteilen verbundene Lösung dieses Problems stellen gesättigte oder ungesättigte Fettalkoholoxethylate mit niedrigen Ethoxylierungsgraden, vorzugsweise mit ca. 2,5 mol EO/mol (A. Behler et al., Seifen, Öle, Fette, Wachse 116, 60 (1990) dar. Nachteilig bei diesem Konzept sind das sehr hohe Fettlösevermögen solcher Oxethylate, ihre eingeschränkte Löslichkeit in wäßrigen Systemen und ihre schaumreduzierende Wirkung. Weiterhin ist ihre Wirksamkeit in praxisnahen Zubereitungen, daß heißt bei realistischen Elektrolytmengen, auf Sulfattenside beschränkt (A. Behler et al. loc. cit.).

Verdickungsmittel für wäßrige Lösungen, die unabhängig vom Tensidtyp wirksam sind, gehören zur Gruppe der wasserlöslichen Polymere. Geeignete Additive sind hierbei Cellulosederivate und Xanthane, bekannt sind auch Polyethylenglycolderivate (DE 31 40 160), Polyolmonoether (EP-0 303 187), mit Fettsäure veresterte Polyoxyalkylenether des Glycerins oder 1.2-Propandiols (DE 32 39 564) oder anderer mehrwertiger Alkohole (DE 38 43 224), Alkylpolyethylenglykoletherfettsäureester (DE 35 41 813), Polyethylenoxid (Polyethylenglykol) allein (EP-A 0 384 983) etc. Die Verdickungswirkung dieser Additive beruht vermutlich darauf, daß ein stark hydratisiertes Netzwerk aufgebaut und das Wasser damit teilweise immobilisiert wird. Zuweilen werden hierbei auch gewisse Synergismen zwischen Tensid und Polymer beobachtet, allerdings ist die zur Einstellung der gewünschten Viskosität notwendige Polymerkonzentration sehr hoch, was einmal zu einer relativ teuren Problemlösung führt und zum anderen ökologisch bedenklich ist, da die Polymere, auch solche auf Polyethylenglycolbasis, nur unzureichend biologisch abbaubar sind. Hinzu kommen bei hohen Polymerkonzentrationen nachteilige Verarbeitungsprobleme und ein zuweilen unbefriedigendes rheologisches Verhalten. Aus alledem folgt, daß Polymere in kosmetischen Zubereitungen möglichst nur in sehr niedrigen Konzentrationen eingesetzt werden sollten.

Höhere Alkohole mit 8 bis 14 Kohlenstoffatomen in Verbindung mit wasserlöslichen anorganischen Salzen bewirken eine bemerkenswerte Minderung der Hautirritation (EP-A 0 388 810) eines neutralen Systems, das als Tensid 3 bis 60 Gew.-% eines (nichtionischen) Alkylglycosids enthält. Die Viskosität kann andeutungsweise durch weitere Zusätze, wie z. B. Tonmineralien oder wasserlösliche Polymeren beeinflußt werden, ohne daß jedoch weitere konkrete Hinweise erfolgen, welche der vielen möglichen Verbindungen damit gemeint sein könnten.

In einer späteren Veröffentlichung des gleichen Anmelders (EP-A 0 408 965) wird ein ähnliches neutrales flüssiges Reinigungsmittel beschrieben, das
a) 2 - 60 Gew.-% eines Alkylpolyglykosids (APG)
b) 0,1 - 10 Gew.-% eines nichtionischen Tensids mit einem HLB-Wert von kleiner als 5,
c) 0,1 - 10 Gew-% eines nichtionischen Tensids mit einem HLB-Wert nicht kleiner als 5 und
d) 0,001 - 8 Gew-.% eines oder mehrerer wasserlöslicher organischer oder anorganischer Salze enthält.

Es wird angedeutet, daß durch Zusatz von wasserlöslichen Polymeren zu diesem System aus drei nichtionischen Tensiden die Viskosität eingestellt werden kann. Es werden jedoch keine Polymeren offenbart.

Ein Konzept, das als Verdickersystem für Tenside gezielt die Kombination Tensid-Polymer ausnützt, wird in DE 38 43 224 beschrieben. Es beansprucht eine Mischung eines nichtionischen Tensids (HLB-Wert von 4 bis 11) mit einem Polymer im Gewichtsverhältnis 10 : 1 bis 1 : 10 als Verdicker für andere Tensidsysteme. Als typisch nichtionische Tenside werden Oxethylate mit niedrigen EO-Graden erwähnt, also genau jene Tenside, deren Verdickerwirkung zwar beachtlich ist, die jedoch sowohl toxikologisch hinsichtlich ihrer Haut und Schleimhautverträglichkeit als auch ökologisch (vgl. P. Schöberl et al., Tenside Surfactants Detergents 25,2 (1988), S. Matsumura JAOCS 67, S. 996 (1990) bedenklich sind.

Es bestand daher die Aufgabe, ein System zur Viskositätserhöhung wäßriger, kosmetischer Tensidzubereitungen, das die oben erwähnten Nachteile nicht aufweist und zu einer toxikologisch, ökologisch sowie ökonomisch befriedigenden Lösung führt, aufzufinden.

Die Aufgabe wurde dadurch gelöst, daß einer tensidischen Basisformulierung als Verdicker eine Mischung aus Tensid, Polymer und gegebenenfalls Elektrolyt hinzugefügt wird.

Gegenstand der Erfindung ist daher eine wäßrige, kosmetische Tensidzubereitung mit erhöhter Viskosität enthaltend
4 bis 30 Gew.-% Basistenside,
3 bis 29,5 Gew.-% Verdickersystem und Additive,
welche dadurch gekennzeichnet sind, daß das Verdickersystem bezogen auf die wäßrige, kosmetische Tensidzubereitung
3 bis 25 Gew.-% Alkylpolyglycosid,
0,005 bis 2 Gew.-% Polymer und
0 bis 2,5 Gew.-% Fremdelektrolyt
enthält.

Es wurde überraschend festgestellt, daß durch die erfindungsgemäß verwendeten Alkylpolyglycoside in Kombination mit geringsten Mengen Polymer und gegebenenfalls geringen Mengen Fremdelektrolyt bei kosmetischen Tensidformulierungen ausreichend hohe Viskositäten erreicht werden.

Bei einem Gehalt an Basistensiden der Formulierung von 4 bis 30 %, vorzugsweise 5 bis 25 %, empfiehlt sich für die Verdickerformulierung:
3 bis 25 %, vorzugsweise 4 bis 20 % Alkylpolyglycoside,
0,005 bis 2 %, vorzugsweise 0,01 bis 1,5 % Polymer,
0 bis 2,5 %, vorzugsweise 0 bis 2 % Fremdelektrolyt.

Basistenside sind in wäßrigen kosmetischen Formulierungen übliche Tenside wie insbesondere Fettalkoholethersulfate, Fettalkoholsulfate, carboxymethylierte Fettalkoholoxethylate, Sulfosuccinate, Alkansulfonate, fettsaure Salze, Betaine, Ampholyte, Fettalkoholoxethylate, Sorbitanester, ethoxylierte Sorbitanester, Zuckerester, sowie deren Gemische.

### Alkylpolyglycoside

Erfindungsgemäß eingesetzte Alkylpolyglycoside genügen der Formen I

R-O-Zₙ I,

in der R für einen linearen oder verzweigten, gesättigten oder ungesättigten aliphatischen Alkylrest mit 10 bis 18 Kohlenstoffatomen oder Gemische davon und Zₙ für einen Polyglycosylrest mit n = 1,1 bis 3 Hexose- oder Pentoseeinheiten oder Gemische davon stehen.

Bevorzugt werden Alkylpolyglycoside mit Fettalkylresten mit 12 bis 16 Kohlenstoffatomen sowie einem Polyglycosylrest von n = 1,1 bis 2,5. Besonders bevorzugt sind Alkylpolyglycoside mit einem HLB-Wert zwischen 11,5 und 17, der z. B. via Emulsionsmethode bestimmt werden kann.

Die erfindungsgemäß eingesetzten Alkylpolyglycoside können nach bekannten Verfahren auf Basis nachwachsender Rohstoffe hergestellt werden. Beispielsweise wird Dextrose in Gegenwart eines sauren Katalysators mit n-Butanol zu Butylpolyglycosidgemischen umgesetzt, welche mit langkettigen Alkoholen ebenfalls in Gegenwart eines sauren Katalysators zu den gewünschten Alkylpolyglycosidgemischen umglycosidiert werden.

Die Struktur der Produkte ist in bestimmten Grenzen variierbar. Der Alkylrest R wird durch die Auswahl des langkettigen Alkohols festgelegt. Günstig aus wirtschaftlichen Gründen sind die großtechnisch zugänglichen Tensidalkohole mit 10 bis 18 C-Atomen, insbesondere native Fettalkohole aus der Hydrierung von Fettsäuren bzw. Fettsäurederivaten. Verwendbar sind auch Ziegleralkohol oder Oxoalkohole.

Der Polyglycosylrest Zₙ wird einerseits durch die Auswahl des Kohlenhydrats und andererseits durch die Einstellung des mittleren Polymerisationsgrades n z. B. nach DE-OS 19 43 689 festgelegt. Im Prinzip können bekanntlich Polysaccharide, z. B. Stärke, Maltodextrine, Dextrose, Galaktose, Mannose, Xylose etc. eingesetzt werden. Bevorzugt sind die großtechnisch verfügbaren Kohlenhydrate Stärke, Maltodextrine und besonders Dextrose. Da die wirtschaftlich interessanten Alkylpolyglycosidsynthesen nicht regio- und stereoselektiv verlaufen, sind die Alkylpolyglycoside stets Gemische von Oligomeren, die ihrerseits Gemische verschiedener isomerer Formen darstellen. Sie liegen nebeneinander mit α- und β-glycosidischen Bindungen in Pyranose- und Furanoseform vor. Auch die Verknüpfungsstellen zwischen zwei Saccharidresten sind unterschiedlich.

Erfindungsgemäß eingesetzte Alkylpolyglycoside lassen sich auch durch Abmischen von Alkylpolyglycosiden mit Alkylmonoglycosiden herstellen. Letztere kann man z. B. nach EP-A-0 092 355 mittels polarer Lösemittel, wie Aceton, aus Alkylpolyglycosiden gewinnen bzw. anreichern.

Der Glycosidierungsgrad wird zweckmäßigerweise mittels 1H-NMR bestimmt.

Im Vergleich zu allen anderen in kosmetischen Reinigungsmitteln eingesetzten Tensiden gelten die Alkylpolyglycoside als überaus umweltverträglich. So liegt der mittels Kläranlagen-Simulationsmodell/DOC-Analyse bestimmte biologische Abbaugrad für die erfindungsgemäßen Alkylpolyglycoside bei 96 ± 3 %. Diese Zahl ist vor dem Hintergrund zu sehen, daß bei diesem Testverfahren (Totalabbau) bereits bei einem Abbaugrad > 70 % die Substanz als gut abbaubar gilt.

Auch die akute orale Toxizität LD 50 (Ratte) sowie die aquatische Toxizität LC 50 (Goldorfe) und EC 50 (Daphnien) mit Werten von > 10 000 mg/kg, 12 bzw. 30 mg/l liegen um den Faktor 3 bis 5 günstiger als die entsprechenden Werte der heute wichtigsten Tenside. Ähnliches gilt für die bei kosmetischen Formulierungen besonders wichtige Haut- und Schleimhautverträglichkeit.

### Polymere

Erfindungsgemäße Polymere sind insbesondere Derivate alkoxylierter, insbesondere ethoxylierter mehrwertiger Alkohole mit 2 bis 8 C-Atomen wie Ethylenglycol, Propandiol, Glycerin, Butandiol, Erythrit, Pentaerythrit, Arabit, Sorbit, etc. Als Derivate bevorzugt sind Fettsäureester und Fettalkoholether, wobei die gesättigten oder ungesättigten, geradkettigen oder verzweigten Alkylreste 8 bis 20 Kohlenstoffatome besitzen können und der mittlere Alkoxylierungsgrad 20 bis 500 mol/mol beträgt.

Weitere bevorzugte Polymere und Fettsäureester von alkoxylierten, insbesondere ethoxylierten Fettalkoholen entsprechen der Formel II

R '-O (C₂H₄O)ₘ COOR'' (II),

worin R' einen geradkettigen (oder evtl. auch verzweigten) gesättigten oder ungesättigten Alkylrest mit 8 bis 20 Kohlenstoffatomen oder Gemische davon, R'' einen geradkettigen (oder evtl. auch verzweigten) gesättigten oder ungesättigten Alkylrest mit 8 bis 20 Kohlenstoffatomen oder Gemische davon und m eine Durchschnittszahl von 20 bis 100 bedeuten.

Schließlich werden als Polymere auch Polyglykolether mit mittleren Molmassen zwischen 2 000 und 25 000 g/mol bevorzugt.

Als Polymere in Frage kommen - sie sind weniger bevorzugt - auch Polymere auf Cellulosebasis oder Xanthan.
Anwendung können auch Polymergemische finden.

### Basistenside

In wäßrigen kosmetischen Formulierungen übliche Tenside wie insbesondere Fettalkoholethersulfate, Fettalkoholsulfate, carboxymethylierte Fettalkoholoxethylate, Fettalkoholethersulfosuccinate, Alkansulfonate, fettsaure Salze, Alkylbetaine, Ampholyte, Fettalkoholoxethylate, Fettsäuresorbitanester, ethoxylierte Sorbitanester, Zuckerester, sowie deren Gemische gelten als Basistenside, wobei die Kettenlänge der gesättigten oder ungesättigten, geradkettigen oder verzweigten Alkylkette jeweils 8 bis 22, vorzugsweise 10 bis 20, Kohlenstoffatome betragt, und die Kationen der anionischen Tenside Na, K, NH₄, C₂-C₃-Alkanolammonium oder Mg sind. Die Ethoxylierungsgrade liegen bei den Fettalkoholethersulfaten zwischen 1 und 5 (vorzugsweise zwischen 2 und 4), bei den carboxylierten Oxethylaten zwischen 2 und 15 (3 und 10), bei den Fettalkoholethersulfosuccinaten zwischen 1 und 6 (2 und 4), bei den Fettalkoholoxethylaten zwischen 2 und 25 (2 und 15) mal Ethylenoxid/mol.

### Elektrolytzusätze

Durch Zusatz von wasserlöslichen Elektrolyten läßt sich die Viskosität der erfindungsgemäßen wäßrigen Tensidzubereitungen für kosmetische Formulierungen gegebenenfalls optimieren, so daß sich in der Regel ihr Zusatz empfiehlt. In Frage kommen hierzu u. a. Alkali-, Ammonium- und Erdalkalihalogenide, -sulfate oder -phosphate.

### Weitere Bestandteile

Die erfindungsgemäßen wäßrigen Zubereitungen können weitere Komponenten enthalten, die für den jeweiligen Anwendungszweck bedeutsam sind. In Frage kommen Silikontenside, Eiweißhydrolysate, Duftstoffe, Trübungs- und Perlglanzmittel, Rückfetter, Silikonöle, Feuchthaltemittel, Konservierungsmittel, hautkosmetische Wirkstoffe, Pflanzenextrakte, Puffersubstanzen, Komplexbildner, etc.

Die folgenden Beispiele sollen die Erfindung erläutern:

### Beispiel 1

5 g carboxymethyliertes C₁₂C₁₄-Fettalkoholoxethylat mit 4 mol EO/mal (Carboxymethylierungsgrad 95 %), 5 g C₁₂C₁₄-Alkylpolyglycosid (Glycosidierungsgrad 1.3, HLB-Wert 13.0) und 0,4 g Antil^{R} 141 solid wurden bei 50 - 60 °C in Wasser gelöst. Es wurde 1 g NaCl hinzugefügt und mit Wasser mit 100 ml aufgefüllt. Die Viskosität der Lösung bei Scherraten von ca. 10 sec⁻¹ betrug 3 500 mPa·s.

### Beispiel 2 (Vergleichsbeispiel)

10 g carboxymethyliertes C₁₂C₁₄-Fettalkoholoxethylat (Carboxymethylierungsgrad 95 %) und 0,4 g Antil^{R} 141 solid wurden wie in Beispiel 1 in Wasser gelöst und mit NaCl versetzt. Die Viskosität - gemessen wie in Beispiel 1 - lag bei 2 mPa·s und war im Vergleich zur reinen Tensidlösung, der 1 % NaCl zugesetzt wurde, durch das Polymer nicht erhöht.

Der Vergleich der Beispiele 1 und 2 zeigt die ungewöhnlich synergistische verdickende Wirkung des Systems Alkylpolyglycosid und Polymer bei bereits niedrigen Elektrolytkonzentrationen im Falle eines Aniontensids, das sich sonst nur schwer verdicken läßt.

### Beispiel 3

Sorbit wurde in Gegenwart von NaOH bei ca. 170 °C ethoxyliert (ca. 300 mol EO/mol) und sodann mit Stearinsäure in Gegenwart von Isopropyltitanat als Katalysator bei ca. 210 °C und 30 mbar verestert. Nach ca. 8 h und Einstellung einer OH-Zahl von ≦ 5 mg KOH/g ist die Reaktion beendet. Das Produkt hat einen Erweichungspunkt von ca. 60 °C.

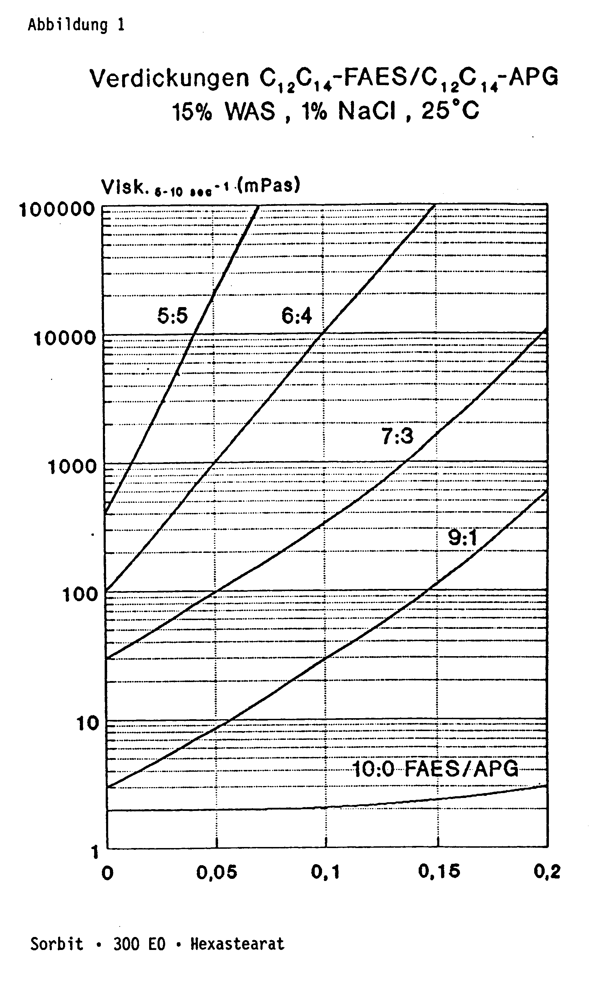

Das so hergestellte Polymer wird nun wäßrigen Lösungen unterschiedlicher Gemische aus C₁₂C₁₄-Fettalkoholethersulfat mit 2 mol EO/mol und C₁₂C₁₄-Alkylpolyglycosid (Glycosidierungsgrad 1.5, HLB-Wert 13.8) bei Gegenwart von 1 % NaCl in verschiedenen Konzentrationen zugesetzt. Die Viskositätsmessung nach ca. 24 h ergibt die in Abbildung 1 dargestellten Resultate.

Während sich reines Fettalkoholethersulfat durch das zugesetzte Polymer im betrachteten Konzentrationsbereich praktisch nicht verdicken läßt, ist die Viskositätserhöhung in Gegenwart des Alkylpolyglycosids extrem stark. Bei 15 % waschaktiver Substanz und einem FAES/APG-Verhältnis von 1 : 1 reichen bereits 0,03 % Polymer, um eine shampoo-typische Viskosität von ca. 5 000 mPa·s zu erzielen. Die erfindungsgemäßen Formulierungen zeigen ohne und in Gegenwart von künstlichem Sebum (30 % Triolein, 20 % Tristearin, 20 % Squalan, 15 % Ölsäure, 10 % Palmitinsäure, 5 % Stearinsäure) im Vergleich mit käuflichen Shampoos hervorragendes Schäumvermögen (Reibschaum nach Wilmsmann, 2 g/l).

### Beispiel 4

0,4 g des Sorbit-Polymers aus Beispiel 3 wurden 7,5 g C₁₂C₁₄-Fettalkoholethersulfat mit 2 mal EO/mal und 7,5 g C₁₂C₁₄-Alkylpolyglycosid mit einem Glycosidierungsgrad von 1.5 (HLB-Wert 13.8) zugesetzt und mit Wasser auf 100 ml ergänzt. Die Viskosität der klaren Lösung - gemessen bei einer Scherrate von ca. 10 sec⁻¹ lag bei ca. 5 000 mPa·s.

Das Beispiel demonstriert, daß shampoo-gemäße Verdickungen bei der erfindungsgemäßen Formulierung bereits durch sehr niedrige Polymerkonzentrationen ohne Fremdelektrolyt erreichbar sind.

### Beispiel 5

526 g LIPOXOL^{R} 4000 (Polyethylenglycol, mittlere Molmasse 4 000), 73,7 g Ölsäure und 0,6 g Isopropyltitanat wurden bei 180 °C im Wasserstrahlvakuum ca. 20 h bis zu einer Säurezahl < 1 mg KOH/g erhitzt.

1 g des so hergestellten Diesters wurde zu 100 ml wäßriger Lösung mit 7,5 % carboxymethylierten C₁₂C₁₄-Fettalkoholoxethylat mit 4 mol EO/mal, 7,5 % C₁₂C₁₄-Alkylpolyglycosid (Glycosidierungsgrad 1.5) und 1 % NaCl gegeben. Die Viskosität dieser Lösung lag bei ca. 5 200 mPa·s; das Schäumvermögen mit und ohne Gegenwart von Sebum ist sehr gut und liegt höher als das verschiedener Marktprodukte.

### Beispiel 6

0,3 % des Sorbit-Polymers von Beispiel 3 wurden zu einer wäßrigen Lösung mit 7,5 % Kokosamidopropylbetain und 7,5 % C₁₂C₁₄-Alkylpolyglycosid (Glycosidierungsgrad 1.3) gegeben, wobei das Betain herstellungsbedingt 2,2 % NaCl enthielt. Die Viskosität der klaren Lösung lag bei 6 000 mPa·s. Im Gegensatz hierzu liegt die Viskosität einer 15%igen Betainlösung bei gleicher Polymerkonzentration und gleichem Elektrolytgehalt bei 4 mPa·s. Der Vergleich der Werte demonstriert die hervorragende konsistenzregulierende Wirkung der erfindungsgemäßen Formulierung.

### Beispiel 7

Zu einer wäßrigen Lösung mit 5 % C₁₂C₁₄-Fettalkoholethoxylatsulfosuccinat und ca. 3 mol EO/mal, 5 % C₁₂C₁₄-Alkylpolyglycosid (Glycosidierungsgrad 1.3) und 1 % NaCl wurden 1 % Antil^{R} solid gegeben. Die Viskosität des entstandenen klaren Gels liegt bei 17 000 mPa·s, gemessen bei einer Scherrate von ca. 10 sec⁻¹.

### Beispiel 8

Zu einer wäßrigen Lösung mit 5 % C₁₂C₁₄-Fettalkoholethersulfat (2 mol EO/mol), 5 % C₁₀C₁₂-Alkylpolyglycosid (HLB-Wert 14.5) und 1 % NaCl werden 0,8 % Sorbit-Polymer (vgl. Beispiel 3) gegeben. Die Viskosität der Lösung liegt bei ca. 4 500 mPa·s, der Klarpunkt liegt bei 2 °C, das Schäumvermögen ist hervorragend.

### Beispiel 9

Zu einer wäßrigen Lösung mit 5 % C₁₂C₁₄-Fettalkoholethersulfat (2 mol EO/mal), 5 % C₁₂C₁₄-Alkylpolyglycosid (Glycosidierungsgrad 1.5) und 1 % NaCl wird 1 % Antil^{R} 141 solid gegeben. Das entstandene Gel besitzt eine Viskosität von ca. 15 000 mPa·s bei einer Scherrate von 10 sec⁻¹ und einen Klarpunkt von 5 °C.

Abbildung 1 zeigt den Viskositätsaufbau mit Hilfe des erfindungsgemäßen Verdickersystems. Mit steigendem Verhältnis von Alkylpolyglycosid/Fettalkoholethersulfat nimmt die Viskosität extrem stark zu. Zur Herstellung geeigneter Viskositäten reichen hier bereits Polymerkonzentrationen ≦ 0,03 %.

## Patentansprüche

1. Wäßrige, 4 bis 30 Gew.-% Basistenside enthaltende kosmetische Tensidzubereitungen mit erhöhter Viskosität,
dadurch gekennzeichnet,
daß zur Viskositätserhöhung ein Verdickersystem eingesetzt wird, das aus einer Kombination aus
3 bis 25 Gew.-% Alkylpolyglycosid und
0,005 bis 2 Gew.-% Polymer und
0 bis 2,5 Gew-% wasserlöslichen anorganischen Fremdelektrolyten besteht, wobei das Polymer
a. ein Fettalkoholether oder Fettsäureester mit geradkettigen oder verzweigten, gesättigten oder ungesättigten C₈-C₂₀-Ketten oder Gemischen von alkoxylierten mehrwertigen Alkoholen mit 2 bis 8 C-Atomen und/oder
b. ein C₈-C₂₀-Fettsäureester von alkoxylierten C₈-C₂₀-Fettalkoholen, wobei die Alkylreste geradkettig oder verzweigt, gesättigt oder ungesättigt bzw. gemischt strukturiert sein können, und/oder
c. ein Polyglykoletherdiester und/oder -ether mit einer mittleren Molmasse zwischen 2 000 und 25 000 g/mol und
d. gegebenenfalls ein Polymer auf Cellulosebasis oder Xanthan ist und wobei
gegebenenfalls weitere Bestandteile enthalten sind.

2. Wäßrige, kosmetische Tensidzubereitungen nach Anspruch 1,
dadurch gekennzeichnet,
daß das Alkylpolyglycosid der Formel I
R-O-Zₙ (I)
entspricht, wobei R für einen linearen oder verzweigten, gesättigten oder ungesättigten Alkylrest mit 8 bis 18 C-Atomen oder Gemische davon und Zₙ für einen Polyglycosidrest mit n = 1 bis 5 Hexose- oder Pentoseeinheiten oder Gemische davon stehen.

3. Wäßrige, kosmetische Tensidzubereitungen nach den Ansprüchen 1 und 2,
dadurch gekennzeichnet,
daß das Alkylpolyglycosid ein Alkylpolyglycosid mit einem HLB-Wert zwischen 11,5 und 17 ist.

4. Wäßrige, kosmetische Tensidzubereitungen nach Anspruch 1,
dadurch gekennzeichnet,
daß das Alkoxylat ein Oxethylat ist und der Ethoxylierungsgrad zwischen 20 und 500 liegt.

5. Wäßrige, kosmetische Tensidzubereitungen nach den Ansprüchen 1 bis 4,
dadurch gekennzeichnet,
daß Polymergemische eingesetzt werden.

## Claims

1. An aqueous, cosmetic surfactant formulation of elevated viscosity containing 4 to 30% by weight of base surfactants, characterised in that a thickener system which comprises a combination of
3 to 25% by weight of alkyl polyglycoside and
0.005 to 2% by weight of polymer and
0 to 2.5% by weight of water-soluble, inorganic foreign electrolytes is used to elevate the viscosity, where the polymer is
a. a fatty alcohol ether or fatty acid ester having straight-chain or branched, saturated or unsaturated C₈-C₂₀ chains or mixtures of alkoxylated polyhydric alcohols having 2 to 8 C atoms and/or
b. a C₈-C₂₀-fatty acid ester of alkoxylated C₈-C₂₀-fatty alcohols, it being possible for the alkyl radicals to be straight-chain or branched, saturated or unsaturated or of mixed structure, and/or
c. a polyglycol ether diester and/or ether having an average molecular mass from 2,000 to 25,000 g/mol and
d. if desired, a cellulose-based polymer or xanthan, and where
further constituents are contained if desired.

2. An aqueous, cosmetic surfactant formulation according to claim 1, characterised in that the alkyl polyglycoside has the formula I
R-O-Zₙ (I)
in which R represents a linear or branched, saturated or unsaturated alkyl radical having 8 to 18 C atoms, or mixtures thereof, and Zₙ represents a polyglycoside radical where n = 1 to 5 hexose or pentose units, or mixtures thereof.

3. An aqueous, cosmetic surfactant formulation according to either of claims 1 and 2, characterised in that the alkyl polyglycoside is an alkyl polyglycoside having an HLB value from 11.5 to 17.

4. An aqueous, cosmetic surfactant formulation according to claim 1, characterised in that the alkoxylate is an oxethylate and the degree of ethoxylation is from 20 to 500.

5. An aqueous, cosmetic surfactant formulation according to any of claims 1 to 4, characterised in that polymer mixtures are used.

## Revendications

1. Préparations tensioactives cosmétiques aqueuses, à haute viscosité, contenant de 4 à 30 % en poids de tensioactifs de base, caractérisées en ce que, pour augmenter la viscosité, on utilise un système épaississant constitué d'une combinaison :
de 3 à 25 % en poids d'un alkylpolyglycoside, et
de 0,005 à 2 % en poids d'un polymère, et
de 0 à 2,5 % en poids d'électrolytes inorganiques étrangers, solubles dans l'eau, ou le polymère est
a) un éther d'un alcool gras ou un ester d'un acide gras avec des chaînes en C₈-C₂₀ droites ou ramifiées, saturées ou insaturées, ou des mélanges, de poly-alcools alcoxylés ayant de 2 à 8 atomes de carbone, et/ou
b) un ester d'un acide gras en C₈-C₂₀ et d'alcools gras en C₈-C₂₀ alcoxylés, où les radicaux alkyle peuvent avoir une structure droite ou ramifiée, saturée ou insaturée, ou mixte, et/ou
c) un polyglycolétherdiester ou -éther ayant une masse moléculaire moyenne de 2000 à 25 000, et
d) éventuellement un polymère à base de cellulose ou de xanthane,
et d'autres constituants étant éventuellement présents.

2. Préparations tensioactives cosmétiques aqueuses selon la revendication 1, caractérisées en ce que l'alkylpolyglycoside correspond à la formule I
R-O-Zₙ (I),
dans laquelle R est un radical alkyle saturé ou insaturé, à chaîne droite ou ramifiée, ayant de 8 à 18 atomes de carbone, ou leurs mélanges, et Zₙ est un résidu polyglycoside dans lequel n = 1 à 5 motifs hexose ou pentose, ou leurs mélanges.

3. Préparations tensioactives cosmétiques aqueuses selon les revendications 1 et 2,
caractérisées en ce que l'alkylpolyglycoside est un alkylpolyglycoside ayant un rapport hydro-lipophile (valeur HLB) compris entre 11,5 et 17.

4. Préparations tensioactives cosmétiques aqueuses selon la revendication 1,
caractérisées en ce que le produit d'alcoxylation est un produit d'éthoxylation, et que le degré d'éthoxylation est compris entre 20 et 500.

5. Préparations tensioactives cosmétiques aqueuses selon les revendications 1 à 4,
caractérisées en ce qu'on utilise des mélanges de polymères.
